# EUROPEAN PATENT APPLICATION

(11) **EP 2 386 241 A2**
(43) Date of publication of application: **16.11.2011**
(21) Application number: 11165028.9
(22) Date of filing: 06.05.2011
(51) Int. Cl.: A61B 1/01, A61B 1/00

(54) **Guide assembly for endoscope**

(30) Priority: 13.05.2010 JP 2010110922
(71) Applicant: Fujifilm Corporation, Minato-ku Tokyo (JP)
(72) Inventor: Yamakawa, Shinichi, Ashigarakami-gun Kanagawa-ken (JP); Iwasaka, Masayuki, Ashigarakami-gun Kanagawa-ken (JP); Ashida, Tsuyoshi, Ashigarakami-gun Kanagawa-ken (JP); Nakamura, Takayuki, Ashigarakami-gun Kanagawa-ken (JP)
(74) Representative: Klunker . Schmitt-Nilson . Hirsch

(57) **Abstract**

A self-propelled type of guide assembly for an endoscope (10) includes a barrel (50, 51, 91, 92, 111, 112, 126, 127) for receiving an elongated tube (13) of the endoscope in an axial direction (A). A rubber ring (77) retains the barrel to the elongated tube in a form with a clearance space (45, 46) defined between the barrel and the elongated tube. The barrel includes a barrel sleeve, having a first inner diameter according to an outer diameter of the elongated tube. An end cup is formed to extend from an end of the barrel sleeve, has a second inner diameter larger than the first inner diameter, for allowing the steering device in the elongated tube to steer. Furthermore, a movable endless track device has an annular surface, movable on an endless track outside the barrel, for pushing a wall of a body cavity for propulsion in the axial direction. A drive roller moves the endless track device relative to the barrel.

## Description

### BACKGROUND OF THE INVENTION

### 1. Field of the Invention

The present invention relates to a guide assembly for an endoscope. More particularly, the present invention relates to a guide assembly capable of causing an endoscope to enter a body cavity smoothly, and keeping a steering device in the endoscope steerable readily.

### 2. Description Related to the Prior Art

An endoscope is used to diagnose a body cavity, such as a large intestine in a gastrointestinal tract. Manipulation of the endoscope is a difficult process, because the large intestine is a tortuous organ in a human body, and some body parts are very changeable in the position in the body, such as a sigmoid colon and a transverse colon. Learning the manipulation of the endoscope of the large intestine requires much experience and time. If a doctor is insufficiently skilled in the manipulation, physical load to the body of a patient will be very large. In view of such a problem, JP-A 2003-339631 discloses a guide assembly for facilitating entry of the endoscope. To this end, the guide assembly bends back the sigmoid colon, and shortens and flattens the same.

The guide assembly of JP-A 2003-339631 includes a retaining device of a sleeve form, and a fin or projection extending backwards with an inclination with respect to an axial direction of an elongated tube of the endoscope. The retaining device fits a portion of the guide assembly on an inner side of a steering device of the endoscope tightly without a gap. An end of the fin or projection is contacted and bent in contact with a wall of the sigmoid colon or the like. The sigmoid colon is bent by use of the end of the fin as an anchor, and is extended substantially straight, so that the elongated tube can be entered in the body cavity more deeply.

U.S. Pat. Nos. 6,971,990 and 7, 736, 300 (corresponding to JP-A 2009-513250) disclose a self-propelled apparatus for propelling the endoscope in the axial direction in the body cavity to facilitate the manipulation even for an unskilled operator or doctor. The self-propelled apparatus of the documents includes a movable endless track device or crawler device or toroidal device. The endless track device is driven to turn around for the endoscope to travel mechanically. Force of propulsion is created by the endless track device contacting a wall of the large intestine, so as to guide the endoscope deeply in the body cavity.

The elongated tube of the endoscope for entry in the body cavity is flexible. The steering device is present at a proximal end of the head assembly of the elongated tube of the endoscope, and keeps the head assembly steerable in a desired direction. In the head assembly, an imaging window is formed for image forming of an object of interest.

According to JP-A 2003-339631, the retaining device fits the guide assembly in the steering device of the endoscope tightly. U.S. Pat. Nos. 6,971,990 and 7,736,300 disclose the self-propelled apparatus in which a support or housing of the endless track device longitudinally extends in the axial direction of the elongated tube. There is a problem in that the steering of the steering device is obstructed by the combined use of the guide assembly or the self-propelled apparatus, and that flexibility of the elongated tube may be lower. Accordingly, the manipulation may be more difficult.

### SUMMARY OF THE INVENTION

In view of the foregoing problems, an object of the present invention is to provide a guide assembly capable of causing an endoscope to enter a body cavity smoothly, and keeping a steering device in the endoscope steerable readily.

In order to achieve the above and other objects and advantages of this invention, a guide assembly for an endoscope having an elongated tube for entry in a body cavity is provided, and includes a barrel, having a lumen for receiving the elongated tube in an axial direction thereof. A retaining device retains the barrel to the elongated tube to provide a clearance space defined between the barrel and the elongated tube.

The elongated tube includes a head assembly, a flexible device, and a steering device disposed between the head assembly and the flexible device. The retaining device is secured to one of the head assembly, the steering device, and a connection point between the steering device and the flexible device.

The barrel includes a barrel sleeve, having a first inner diameter according to an outer diameter of the elongated tube. At least one large diameter portion is formed to extend from an end of the barrel sleeve, has a second inner diameter larger than the first inner diameter, for allowing the steering device in the elongated tube to steer.

The large diameter portion is formed to extend from at least one of distal and proximal ends of the barrel sleeve in the axial direction.

The second inner diameter in the large diameter portion increases in a direction away from the barrel sleeve.

The large diameter portion extends from the distal end of the barrel sleeve in the axial direction.

Preferably, the at least one large diameter portion includes a first end cup formed to extend from the distal end of the barrel sleeve. A second end cup is formed to extend from the proximal end of the barrel sleeve.

Furthermore, a bearing structure is disposed between the barrel and the retaining device, for connection thereof in a relatively rotatable manner.

The bearing structure includes a bearing ball.

Preferably, the bearing structure includes a bearing support ring disposed between the barrel and the retaining device. A first bearing ball is disposed between the barrel and the bearing support ring. A second bearing ball is disposed between the retaining device and the bearing support ring.

Furthermore, a spacer is contained between the retaining device and the elongated tube, for maintaining the clearance space between the barrel and the elongated tube.

The spacer includes a spacer ring, and at least one gap is formed in the spacer ring to open in the axial direction.

The spacer is shaped in a C form.

Furthermore, a collet chuck device is formed with a distal end of the barrel. The retaining device is fitted around the collet chuck device for retention.

The retaining device is an elastic retaining ring.

Preferably, the retaining device includes a male thread formed around the collet chuck device. A nut has a female thread engaged helically with the male thread and secured to the collet chuck device.

Furthermore, a movable endless track device has an annular surface, disposed to extend outside the barrel, movable on an endless track, for pushing a wall of a body cavity for propulsion in the axial direction. A driving device moves the endless track device on the endless track relative to the barrel.

The driving device includes a first drive roller, having engagement teeth, supported on the barrel, rotatable about an axis extending transversely to the axial direction, for moving the endless track device. A rotating mechanism rotates the first drive roller.

The retaining device is disposed close to the first drive roller.

The rotating mechanism includes a first worm gear, disposed concentrically with the barrel in a rotatable manner, for rotating the first drive roller in mesh therewith.

The rotating mechanism further includes a control wire, disposed to extend from the barrel in a proximal direction along the elongated tube, for transmitting driving force in a distal direction upon being rotated on a proximal side, to rotate the first worm gear.

Furthermore, a guide portion is secured to the elongated tube, has a hole for receiving entry of the control wire, for guiding the control wire along the elongated tube.

The barrel includes a first support barrel for supporting the endless track device movably. A second support barrel is mounted in the first support barrel, and secured to the elongated tube with the retaining device.

Furthermore, a second drive roller has engagement teeth, disposed on a proximal side in the axial direction from the first drive roller, supported on the barrel, rotatable about an axis extending transversely to the axial direction, for moving the endless track device. A second worm gear is formed with the first worm gear on the proximal side in the axial direction, for rotating the second drive roller in mesh therewith.

The rotating mechanism further includes a pinion, disposed on a proximal side from the barrel, and rotated by a distal end of the control wire. Plural spur gear teeth are formed at a proximal end of the first worm gear concentrically, meshed with the pinion, and caused to rotate by rotation of the control wire.

Consequently, it is possible with a guide assembly to cause an endoscope to enter a body cavity smoothly, and keep a steering device in the endoscope steerable readily, because of the use of the retaining device to maintain the clearance space.

### BRIEF DESCRIPTION OF THE DRAWINGS

The above objects and advantages of the present invention will become more apparent from the following detailed description when read in connection with the accompanying drawings, in which:
Fig. 1 is a plan, partially broken, illustrating an endoscope system;
Fig. 2 is a perspective view illustrating an endoscope and a self-propelled type of guide assembly associated with the endoscope;
Fig. 3 is a vertical section illustrating the guide assembly;
Fig. 4 is an exploded perspective view illustrating a driving device with a rotating mechanism;
Fig. 5 is a vertical section illustrating one preferred guide assembly including a second drive roller;
Fig. 6 is a vertical section illustrating another preferred guide assembly with a short type of support barrels;
Fig. 7 is a vertical section illustrating one preferred guide assembly with an end cup on a distal side;
Fig. 8 is a vertical section illustrating still another preferred guide assembly with end cups on proximal and distal sides;
Fig. 9 is a vertical section, partially broken, illustrating one preferred guide assembly with bearing balls;
Fig. 10 is a perspective view illustrating another preferred guide assembly with two groups of bearing balls;
Fig. 11 is an exploded perspective view illustrating a preferred structure for retaining a guide assembly with the endoscope;
Fig. 12 is a perspective view illustrating a preferred example with a guide ring in place of an overtube.

### DETAILED DESCRIPTION OF THE PREFERRED EMBODIMENT(S) OF THE PRESENT INVENTION

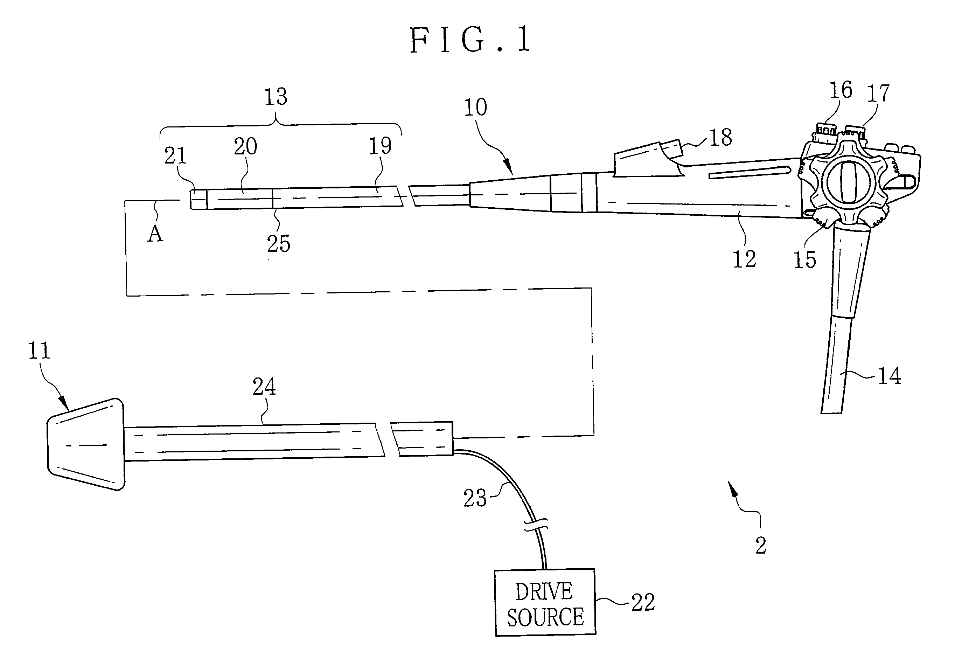

In Fig. 1, an electronic endoscope system 2 includes an electronic endoscope 10 and a self-propelled type of guide assembly 11. The endoscope 10 includes a handle device 12, and an elongated tube 13 or guide tube extending from the handle device 12 for entry in a body cavity, for example, large intestine of a gastrointestinal tract. A universal cable 14 is connected with the handle device 12. Connectors or plugs are provided at an end of the universal cable 14 for connection with a light source apparatus (not shown) and a processing apparatus (not shown).

The handle device 12 includes a steering wheel 15 for bending, an air/water button 16 and a suction button 17. The air/water button 16 is operable for supply of air or water through a distal end of the elongated tube 13. An instrument channel 18 is formed in the elongated tube 13 of the handle device 12 for receiving entry of a medical instrument such as an electrosurgical instrument.

The elongated tube 13 includes a flexible device 19, a steering device 20 and a head assembly 21 in a sequence in a distal direction from the handle device 12. The flexible device 19 has a length as great as several meters for reach of the head assembly 21 to an object of interest in a body cavity. The steering device 20 bends up and down and to the right and left in response to operation of the steering wheel 15 of the handle device 12. Thus, the head assembly 21 can be steered in a desired direction in the patient's body.

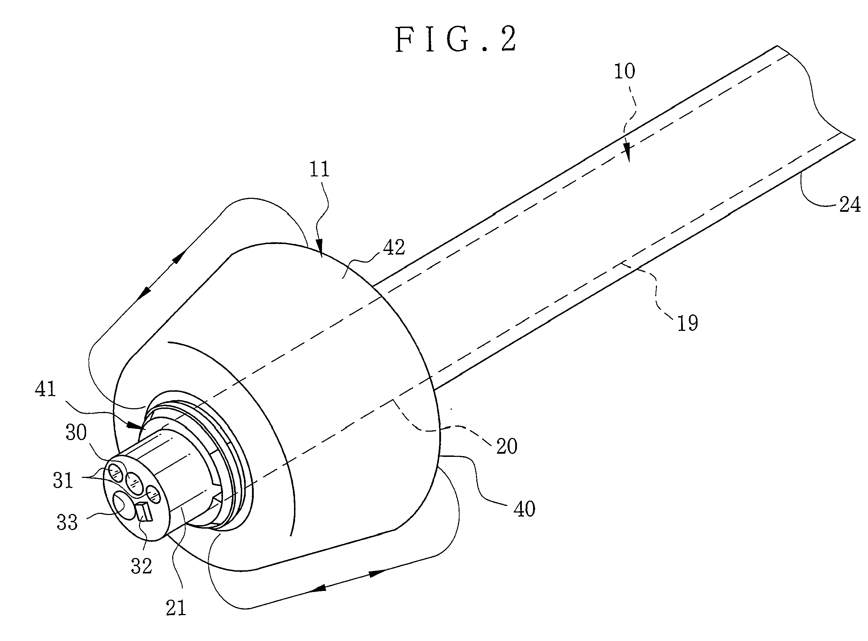

An imaging window 30 is formed in the head assembly 21 for imaging of a body part in the body. See Fig. 2. The head assembly 21 contains objective optics and an image sensor or solid-state image pickup device for imaging, such as CCD and CMOS image sensors. The image pickup device is connected to the processing apparatus by a signal line, which extends through the elongated tube 13, the handle device 12 and the universal cable 14. An object image of the body part is focused on a reception surface of the image pickup device, and is converted into an image signal. The processing apparatus processes the image signal from the image pickup device through the signal line by image processing, and obtains a video signal by conversion after the image processing. The object image is output and displayed on a monitor display panel (not shown) according to the video signal.

Various openings are formed in the head assembly 21 as illustrated in Fig. 2. Among those, a lighting window 31 passes illumination light from a light source apparatus toward the object of interest. An air/water nozzle 32 supplies air or water toward the imaging window from an air/water supply device in the light source apparatus in response to depression of the air/water button 16. An instrument opening 33 causes a distal end of a medical instrument from the instrument channel 18 to appear distally.

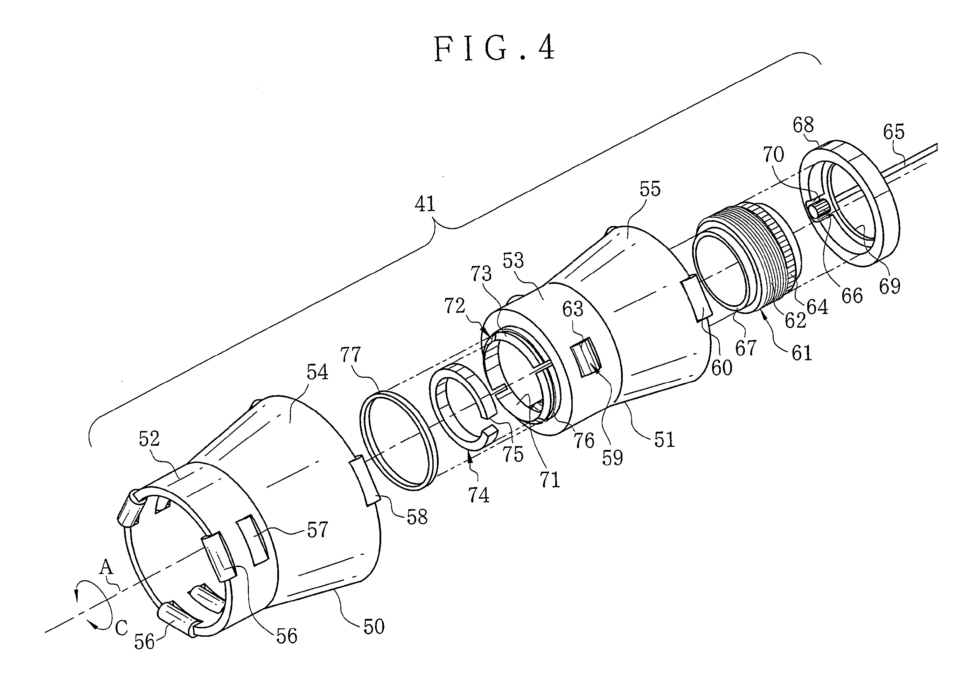

The self-propelled type of guide assembly 11 is used with the endoscope 10 for assistance in moving the elongated tube 13 of the endoscope 10 in and out in through a body cavity. A drive source 22 or motor drives the guide assembly 11. A control wire 65 or torque wire of Fig. 4 is connected with the drive source 22, and transmits torque of rotation for driving the guide assembly 11. A protection sheath 23 extends with a full length of the control wire 65, and receives the control wire 65 for protection. The control wire 65 rotates in the protection sheath 23 when the drive source 22 is driven.

An overtube 24 is used to cover the elongated tube 13, and is ready to expand and shrink in an axial direction A of the elongated tube 13. The protection sheath 23 of the control wire 65 is entered between the overtube 24 and the elongated tube 13.

A controller (not shown) controls the drive source 22. A button panel (not shown) is connected to the controller. The button panel includes a command button for inputting command signals for forward movement, backward movement and stop of the self-propelled type of guide assembly 11, and a speed button for changing a moving speed of the guide assembly 11. Note that a control program can be prepared suitably for an object to be imaged. The drive source 22 can be actuated according to the control program without manipulating the button panel, so as to actuate the guide assembly 11 automatically.

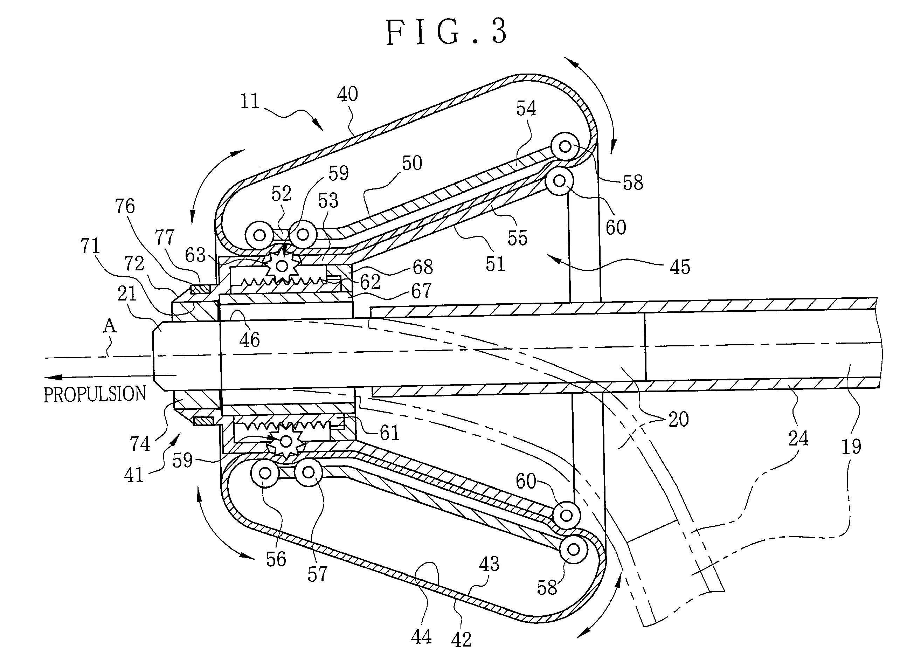

In Fig. 3, the self-propelled type of guide assembly 11 is viewed in a section after division with an interval of 120 degrees. In Figs. 2, 3 and 4, the guide assembly 11 includes a movable endless track device 40 or crawler device or toroidal device, and a driving device 41 or support device or barrel device with a rotating mechanism. The endless track device 40 has a hollow shape with an annular surface, is movable on an endless track, and is formed from a biocompatible plastic material, such as polyvinyl chloride, polyamide resin, fluorocarbon resin and the like. The endless track device 40 includes an outer surface 42 and an inner surface 43.

The outer surface 42 of the endless track device 40 contacts inner surfaces of a body cavity, and exerts force of propulsion of the elongated tube 13 in an axial direction A. As indicated by the arrow, the endless track device 40 turns around in the axial direction A.

To propel the elongated tube 13 in a distal direction, the endless track device 40 in contact with a wall of a body part is moved in a proximal direction reverse to the distal direction. A portion of the endless track device 40 is turned at a proximal end of the self-propelled type of guide assembly 11 with 180 degrees, and bent back into the driving device 41. The portion moves in the distal direction in the internal track, and subsequently is turned over at a distal end of the guide assembly 11 with 180 degrees, and bent back outside the driving device 41. Therefore, the elongated tube 13 is moved in the distal direction by the turn around of the endless track device 40 in an endless manner to orient its outer portion in the proximal direction and its inner portion in the distal direction. To move the elongated tube 13 in the proximal direction, the endless track device 40 is turned around endlessly to orient its outer portion in the distal direction and its inner portion in the proximal direction.

In Fig. 4, the driving device 41 is illustrated. The endless track device 40 is not shown. The driving device 41 has a first support barrel 50 and a second support barrel 51. There is a hollow space 44 in the endless track device 40. The first support barrel 50 is disposed in the hollow space 44. The second support barrel 51 is disposed between the elongated tube 13 of the endoscope 10 and the outer surface 42 of the endless track device 40.

Specifically, the endless track device 40 is prepared in the following manner. At first, a plastic tube having two open ends with flexibility and elasticity is initially formed from a sheet or film of the above-described suitable material. The plastic tube is halfway inserted in a sleeve lumen of the first support barrel. Then a portion of the plastic tube outside the sleeve lumen is bent back externally and extended to cover the periphery of the first support barrel. A first side line of the inserted half of the plastic tube is opposed to a second side line of the bent half to the plastic tube, so that the halves are attached together along the first and second side lines by adhesion, welding or other suitable method. Finally, the toroidal shape of the endless track device 40 is obtained.

The first and second support barrels 50 and 51 have barrel sleeves 52 and 53 and end cups 54 and 55 or skirts or large diameter portions, and are shaped with an equal length. The end cups 54 and 55 are formed with proximal ends of respectively the barrel sleeves 52 and 53, and shaped conically with an increasing diameter in the proximal direction in the self-propelled type of guide assembly 11. A clearance space 45 is defined by the end cups 54 and 55 for smoothing steering of the steering device 20 of the endoscope 10 within the driving device 41.

Idler rollers 56, 57 and 58 or driven rollers are disposed on a peripheral surface of the first support barrel 50 and arranged in the axial direction A. The idler roller 56 is constituted by three rollers arranged about the first support barrel 50 at an angular interval of 120 degrees. The idler rollers 57 and 58 are arranged similarly. The idler roller 56 is secured to a distal end of the barrel sleeve 52 of the first support barrel 50. The idler roller 57 is secured to a proximal end of the barrel sleeve 52. The idler roller 58 is secured to a proximal end of the end cup 54. The idler rollers 56-58 contact the inner surface 43 of the endless track device 40, and are rotated by turn around of the endless track device 40.

A drive roller 59 or toothed roller or worm wheel, and an idler roller or driven roller 60 are arranged on a surface of the second support barrel 51, and rotatably secured thereto. The drive roller 59 is constituted by three rollers arranged in a circumferential direction at an interval of 120 degrees. The idler roller 60 is constituted by three rollers arranged similarly. The drive roller 59 is positioned in the middle of the barrel sleeve 53 of the second support barrel 51. The idler roller 60 is positioned at a proximal end of the end cup 55.

The drive roller 59 is disposed between the idler rollers 56 and 57 of the first support barrel 50 in an assembled state of the self-propelled type of guide assembly 11 so that the endless track device 40 is movable between the drive roller 59 and the idler rollers 56 and 57. The idler roller 60 is opposed to the idler roller 58 of the first support barrel 50, and keeps the endless track device 40 movable between this and the idler roller 58. The drive roller 59 and the idler roller 60 contact the outer surface 42 of the endless track device 40. A worm drive 61 rotates the drive roller 59 to turn around the endless track device 40. The idler roller 60 is rotated by movement of the endless track device 40.

It is preferable to coat surfaces of the elements with lubricant for higher smoothness, namely surfaces of the idler rollers 56-58, the inner surface 43 of the endless track device 40, surfaces of the drive roller 59 and the idler roller 60, and the outer surface 42 of the endless track device 40. Furthermore, a portion of the endless track device 40 for contacting the rollers can be formed from other material having higher smoothness and higher resistance to abrasion.

The worm drive 61 is contained in the barrel sleeve 53 of the second support barrel 51. In the axial direction A, a size of the worm drive 61 is slightly smaller than a size of the barrel sleeve 53. The worm drive 61 is in a symmetric form rotationally about the axis of the axial direction A. A worm gear 62 is disposed on the worm drive 61 helically about the axis as worm gear teeth (threads) . Engagement teeth 63 of the drive roller 59 are meshed with the worm gear 62. Rotation of the worm drive 61 about the axis, namely in the circumferential direction C, is transmitted to the drive roller 59. Preferably, lubricant is applied to the worm gear 62.

Spur gear teeth 64 are formed with a proximal end of the worm drive 61, and arranged in the circumferential direction C. A pinion 66 is connected with the control wire 65, and meshed with the spur gear teeth 64. The pinion 66 is rotated by the control wire 65. The spur gear teeth 64 transmit the rotation of the pinion 66 to the worm drive 61, which rotates in the circumferential direction C.

There is a holding sleeve 67 receiving insertion of the worm drive 61. An edge of the holding sleeve 67 is fitted on an inner surface of a distal end of the barrel sleeve 53 of the second support barrel 51. The elongated tube 13 of the endoscope 10 is introduced in the holding sleeve 67. The holding sleeve 67 has a smaller diameter than an inner diameter of the barrel sleeve 53 of the second support barrel 51 for the purpose of keeping a space for disposing the drive roller 59 and the worm drive 61. The holding sleeve 67 has a larger diameter than an outer diameter of the elongated tube 13 for the purpose of keeping a clearance space 46 (See Fig. 3) between an inner surface of the holding sleeve 67 and an outer surface of the elongated tube 13.

A rear frame 68 is fitted on a proximal end of the holding sleeve 67, and keeps the worm drive 61 rotatable around the holding sleeve 67, and keeps the worm drive 61 and the holding sleeve 67 contained in the barrel sleeve 53. An outer diameter of the rear frame 68 is equal to an inner diameter of the barrel sleeve 53. An opening 69 is formed in the rear frame 68, and has a diameter equal to an outer diameter of the holding sleeve 67. A cutout 70 is formed in the rear frame 68, and contains the pinion 66 in a rotatable manner. A distal end of the control wire 65 is inserted in a hole (not shown) formed in the rear frame 68, and connected with the pinion 66.

Thus, the worm drive 61 is driven by the pinion 66 and rotates about the axis of the axial direction A. The drive roller 59 is rotated by the worm gear 62 of the worm drive 61. In response, the endless track device 40 turns around in a forward or backward direction according to one of rotational directions of the pinion 66 or the worm drive 61.

A retaining device 72 is disposed at a distal end of the barrel sleeve 53 of the second support barrel 51. A lumen 71 is formed through the barrel sleeve 53, and has a diameter slightly smaller than an outer diameter of the holding sleeve 67. A size of the retaining device 72 in the axial direction A is sufficiently smaller than the barrel sleeve 53. A collet portion 73 or collet chuck device with plural collet segments or ridge segments constitutes the retaining device 72, and protrudes from an edge of the lumen 71 distally in the axial direction A. The collet segments in the collet portion 73 have an inclined surface with an inclination in an inward direction toward the distal side, and resiliently shift toward open and closed positions perpendicularly to the axial direction A.

A spacer 74 is a C ring, which is defined by a circular ring and a gap 75 formed in the ring to increase and decrease the crosswise size of the ring. An initial diameter of the spacer 74 is determined in consideration of an outer diameter of the elongated tube 13 of the endoscope 10 for fitting of the self-propelled type of guide assembly 11. As the spacer 74 is deformable, plural types of the endoscope 10 for use with the guide assembly 11 can be combined by absorbing differences in the outer diameter of the elongated tube 13 even with the spacer 74 only.

The spacer 74 is secured to the inside of the retaining device 72 removably. To set the self-propelled type of guide assembly 11 on the elongated tube 13 of the endoscope 10, at first the holding sleeve 67, the spacer 74 and the retaining device 72 are caused to receive entry of the elongated tube 13. In a groove 76 which is formed in the retaining device 72, a rubber ring 77 or retaining ring is fitted. The collet portion 73 and the spacer 74 are firmly clamped by the rubber ring 77 in the inward direction, so that the guide assembly 11 is retained tightly on one connection portion of the elongated tube 13 of the endoscope 10, namely the head assembly 21 according to the embodiment.

Note that the connection portion of the elongated tube 13 as viewed in the axial direction has a size sufficient for keeping a stably retained state, for keeping flexibility of the elongated tube 13 of the endoscope 10, and for smoothing the bend of the steering device 20. The size is preferably equal to or more than 5 mm and equal to or less than 15 mm.

The operation of the endoscope system 2 is described now. At first, the overtube 24 is set to cover the elongated tube 13 of the endoscope 10. The self-propelled type of guide assembly 11 is fitted on the head assembly 21 by use of the retaining device 72. To mount the guide assembly 11, the head assembly 21 is inserted in the holding sleeve 67, the spacer 74 and the retaining device 72 in a sequence. Then the rubber ring 77 is fitted in the groove 76 of the retaining device 72.

After the overtube 24 and the self-propelled type of guide assembly 11 are positioned and attached, the processing apparatus, light source apparatus and control apparatus are turned on for powering. Patient information and other required information is input. Then the elongated tube 13 of the endoscope 10 is entered in a body cavity of a patient's body.

After the head assembly 21 is advanced to a predetermined body part, for example, slightly short of a sigmoid colon, then the button panel is operated to turn on a power source for the drive source 22 of the self-propelled type of guide assembly 11. Then a command signal for start is input with the button panel. The drive source 22 rotates the control wire 65 in a predetermined direction, so that rotation of the pinion 66 with the control wire 65 causes the worm drive 61 to rotate. Its rotation is transmitted to the drive roller 59 to turn around the endless track device 40. An outer portion of the endless track device 40 contacts a wall of a body cavity, to exert force of propulsion in the axial direction. The endless track device 40 of the guide assembly 11 pushes the wall of the body cavity from a distal side toward a proximal side, to propel the head assembly 21 in the distal direction along the wall.

When a command signal for a change is input by operating the button panel, the drive source 22 changes a rotational speed of the control wire 65. Thus, a moving speed of the self-propelled type of guide assembly 11 is changed. When a command signal for return is input by operating the button panel, the drive source 22 causes the control wire 65 to rotate in a backward direction, to move the guide assembly 11 and the head assembly 21 backwards. When a command signal for a stop is input by operating the button panel, the drive source 22 stops to stop moving the guide assembly 11. It is possible to propel the head assembly 21 through the body cavity to an object of interest by suitably repeating those steps of the movement.

The operator rotates the steering wheel 15 to steer the steering device 20 of the endoscope 10, and orients the head assembly 21 in a desired direction. The self-propelled type of guide assembly 11 is firmly retained on one portion of the elongated tube 13 of the endoscope 10. The clearance space 45 is maintained within the end cup 55 of the second support barrel 51. The clearance space 46 is maintained between the holding sleeve 67 and the spacer 74. Thus, it is possible in Fig. 3 to steer the steering device 20 smoothly similarly to a state without the guide assembly 11, because the guide assembly 11 does not obstruct steering of the steering device 20. If the elongated tube 13 remains straight, the guide assembly 11 contacts the elongated tube 13 only at the retaining device 72. This is effective in keeping high flexibility of the elongated tube 13 even in retaining the guide assembly 11.

The disposition, numbers and the like of the idler rollers and drive rollers are not limited to the above embodiment. For example, the idler rollers 58 and 60 may be provided on the barrel sleeves 52 and 53 of the first and second support barrels 50 and 51. The idler rollers 56 and 57 and the drive roller 59 can be provided on the end cups 54 and 55. However, a close disposition of the retaining device 72 to the drive roller 59 is preferable because vibration with the drive roller 59 can be suppressed.

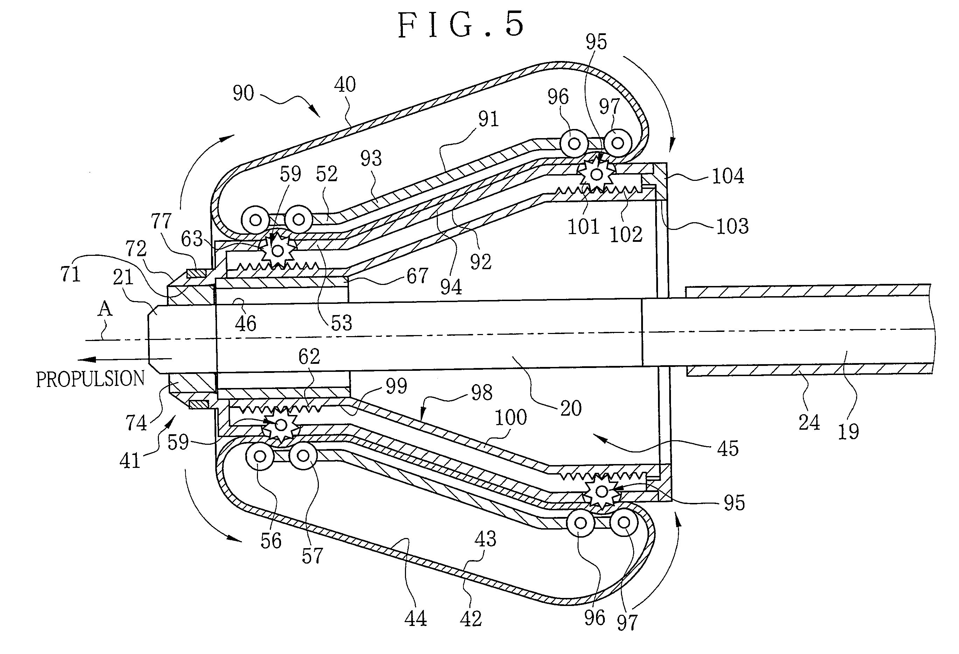

In Fig. 5, another preferred self-propelled type of guide assembly 90 is illustrated. A first support barrel 91 has an end cup 93 or skirt. A second support barrel 92 has an end cup 94 or skirt. A drive roller 95 or toothed roller or worm wheel is disposed at a proximal end of the end cup 94 in place of the idler roller 60. Idler rollers 96 and 97 or driven rollers are disposed on the end cup 93 and opposed to the drive roller 95. In combination with this structure, a worm drive 98 includes a sleeve 99 and a cup 100 or skirt in a manner near to the first and second support barrels 91 and 92. The worm gear 62 is formed on the sleeve 99 for mesh with the engagement teeth 63 of the drive roller 59. A second worm gear 102 is formed on the cup 100 as worm gear teeth (threads) for mesh with engagement teeth 101 of the drive roller 95. A rear frame 104 is fitted on proximal ends of the end cup 94 and the cup 100 of the second support barrel 92 and the worm drive 98. The rear frame 104 has an outer diameter equal to that of a proximal end of the end cup 94. An opening 103 is formed in the rear frame 104, and has a diameter equal to an outer diameter of a proximal end of the cup 100 of the worm drive 98. Elements similar to those of the above embodiment are designated with identical reference numerals.

Furthermore, it is possible to operate the roller 59 as an idler roller and use only the drive roller 95 for driving, instead of the drive rollers 59 and 95 in the embodiment of Fig. 5. In short, the worm gear 62 in the worm drive 98 can be omitted so that a cylindrical surface is present instead. When the worm drive 98 rotates about the axis of the axial direction A, the drive roller 95 rotates to turn around the endless track device 40. The roller 59 is caused to rotate together with the idler rollers 56 and 57.

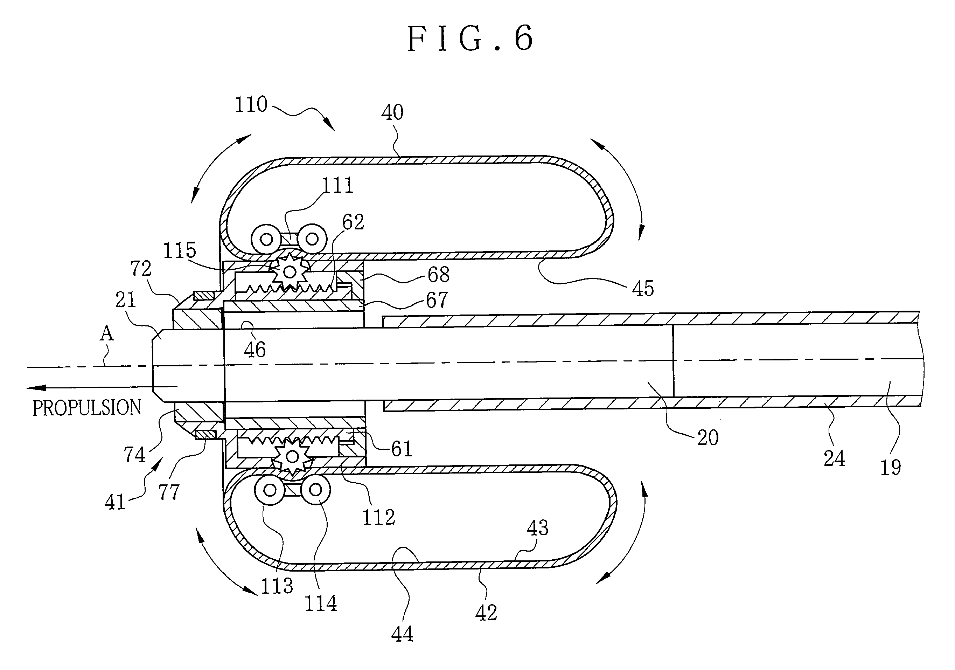

In Fig. 6, a further preferred self-propelled type of guide assembly 110 without an end cup is illustrated. A first support barrel 111 and a second support barrel 112 are cylindrical. A drive roller 115 or toothed roller or worm wheel is rotatable on the second support barrel 112. Idler rollers 113 and 114 or driven rollers are rotatable on the first support barrel 111. A proximal end of the endless track device 40 is not supported but extends in a free manner with the clearance space 45. The material of the endless track device 40 is a flexible compound, so that the proximal end of the endless track device 40 is deformed resiliently upon steering of the steering device 20 without blocking the steering. Thus, the use of the guide assembly 110 of Fig. 6 can achieve effects similar to the above embodiment. It is possible to reduce costs for parts and a manufacturing cost in comparison with the above embodiment, because of absence of the end cup and the rollers at the end cup. Also, it is possible to fill the hollow space 44 of the endless track device 40 with fluid of a biocompatible property, such as water, nitrogen gas or the like.

In the above embodiments, the head assembly 21 is used for the retention of the self-propelled type of guide assembly 11. However, a position of the retention of the guide assembly 11 may be determined in other manners suitably, for example, in the steering device 20, or at a connection point 25 or interface between the steering device 20 and the flexible device 19 as illustrated in Fig. 1.

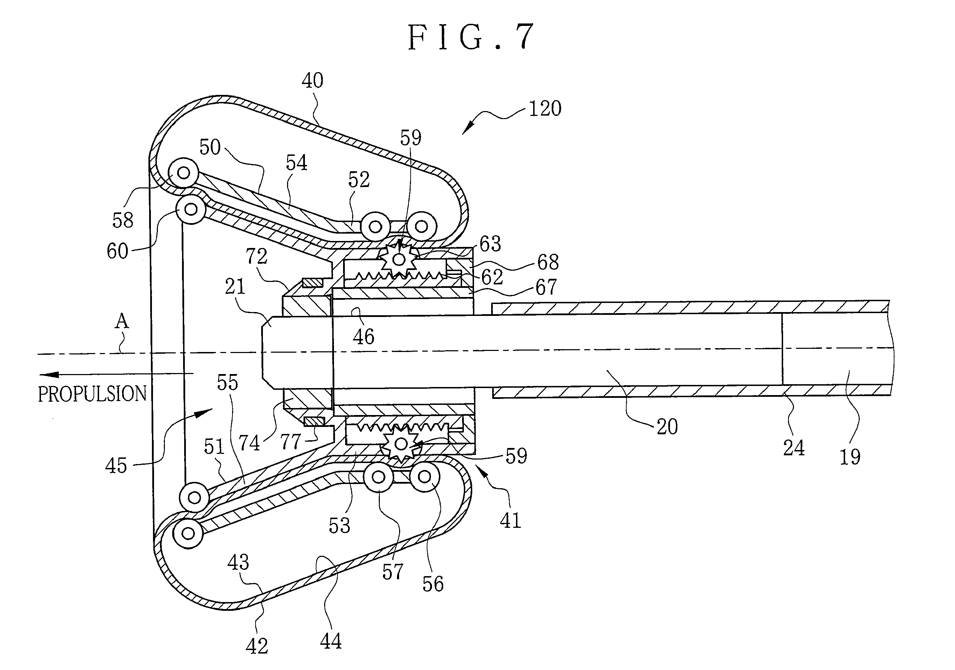

In the above embodiment, the end cups 54 and 55 are disposed on a proximal side of the elongated tube 13. In contrast, in Fig. 7, an additional preferred self-propelled type of guide assembly 120 is illustrated, in which the end cups 54 and 55 are disposed on a distal side of the elongated tube 13. Except for the feature of the end cups 54 and 55, the structure of the guide assembly 120 is basically the same as the guide assembly 11. Elements similar to those of the guide assembly 11 are designated with identical reference numerals.

As the clearance space 45 in the self-propelled type of guide assembly 11 in the first embodiment is oriented on a proximal side of the elongated tube 13, unwanted imaging of the endless track device 40 in a field of view of the endoscope 10 is prevented. A profile of the entirety of the elongated tube 13 including the guide assembly 11 is in a form of decreasing its width, so that the entry of the elongated tube 13 in a body cavity can be smooth. If the clearance space 45 in the guide assembly 120 is oriented on a distal side of the elongated tube 13 as illustrated in Fig. 7, the outer surface 42 of the endless track device 40 contacts a wall of a body cavity in a position short of that according to the first embodiment. Thus, force of propulsion of the guide assembly 120 can be high. Note that for the structure of Fig. 7, a point of the retention of the guide assembly is determined to prevent unwanted imaging of the endless track device 40 in a field of view of the endoscope 10.

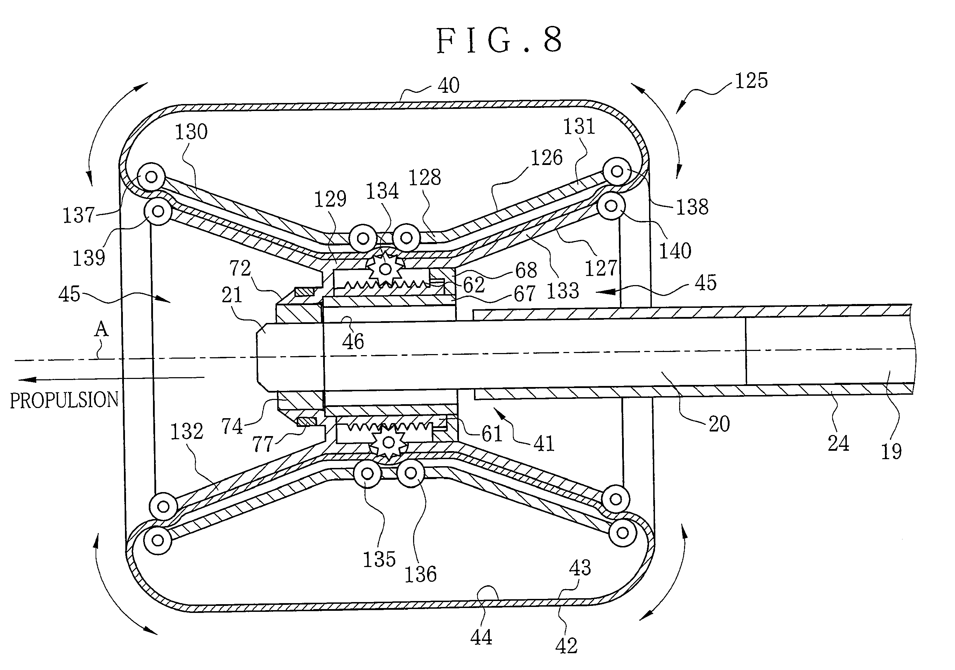

In Fig. 8, still another preferred self-propelled type of guide assembly 125 is illustrated. The guide assembly 125 includes a first support barrel 126 and a second support barrel 127 of a two-cup structure. The first support barrel 126 includes a barrel sleeve 128, and skirts 130 and 131 or end cups or large diameter portions formed to project from respectively ends of the barrel sleeve 128. The second support barrel 127 includes a barrel sleeve 129, and skirts 132 and 133 or end cups or large diameter portions formed to project from respectively ends of the barrel sleeve 129. A drive roller 134 or toothed roller or worm wheel is supported on the barrel sleeve 129 in a rotatable manner. Idler rollers 135 and 136 or driven rollers are supported on the barrel sleeve 128 in a rotatable manner, and opposed to the drive roller 134. Idler rollers 137 and 138 or driven rollers are supported on ends of the skirts 130 and 131 in a rotatable manner. Idler rollers 139 and 140 or driven rollers are supported on ends of the skirts 132 and 133 in a rotatable manner. The guide assembly 125 is characterized in that the clearance space 45 is present in each of the front and rear sides of the elongated tube 13, so that the degree of freedom in movement of the endoscope 10 can be higher.

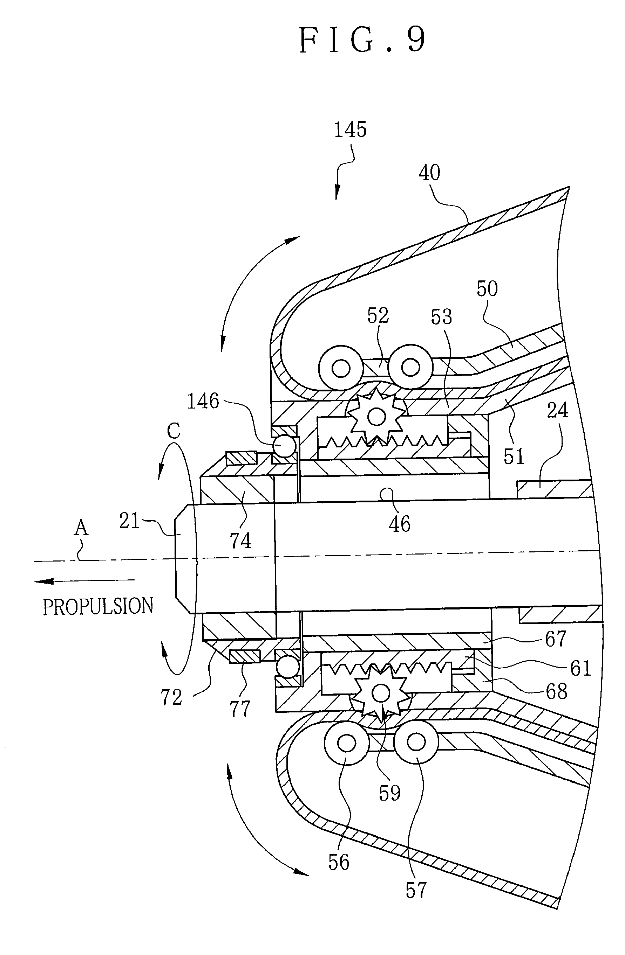

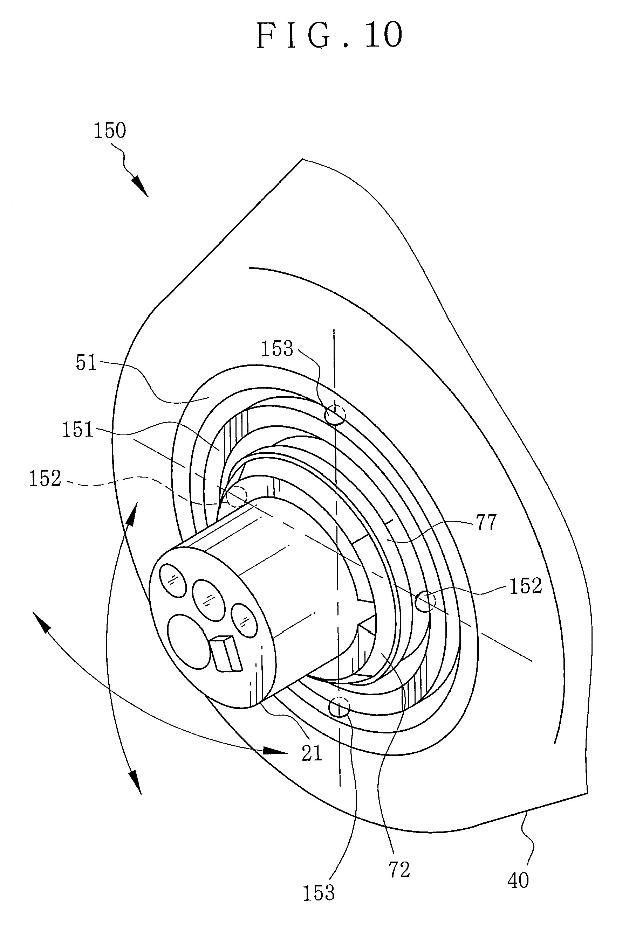

Also, it is possible to construct a self-propelled type of guide assembly in manners of Figs. 9 and 10 for higher degree of freedom of the endoscope 10 in a body cavity. In Fig. 9, a self-propelled type of guide assembly 145 includes the second support barrel 51 separate from the retaining device 72 of the guide assembly 11. Bearing balls 146 are disposed between the retaining device 72 and the second support barrel 51 for connection. For example, the four bearing balls 146 are arranged circumferentially at an interval of 90 degrees. Remaining portions of the guide assembly 145 are the same as those of the guide assembly 11.

The bearing balls 146 keep the retaining device 72 rotatable relative to the second support barrel 51 in the circumferential direction C. Thus, the elongated tube 13 of the endoscope 10 fitted on the retaining device 72 is rotatable in the circumferential direction C. When a proximal portion of the elongated tube 13 near to the handle device 12 is rotated in the circumferential direction C, the retaining device 72 rotates relative to the second support barrel 51, to change the view of imaging easily during manipulation. If distortion occurs in the elongated tube 13 in the circumferential direction C, the retaining device 72 responsively rotates to cancel the distortion. This is effective in preventing occurrence of excessive stress to the elongated tube 13.

In Fig. 10, a self-propelled type of guide assembly 150 is illustrated. The retaining device 72 is separate from the second support barrel 51. A bearing support ring 151 in a bearing structure is disposed between the retaining device 72 and the second support barrel 51. Bearing balls 152 are disposed between the retaining device 72 and the bearing support ring 151. Bearing balls 153 are disposed between the bearing support ring 151 and the second support barrel 51. The bearing support ring 151 has a size equal to that of the retaining device 72 in the axial direction. The bearing balls 152 are two arranged in opposite positions in the circumferential direction. The bearing balls 153 are two arranged similarly. Remaining portions of the guide assembly 150 are the same as those of the guide assembly 11.

The bearing balls 152 keep the retaining device 72 rotatable circumferentially and in a vertical direction in the drawing relative to the bearing support ring 151. Also, the bearing balls 153 keep the bearing support ring 151 rotatable in the horizontal direction relative to the second support barrel 51. Thus, the elongated tube 13 of the endoscope 10 secured to the retaining device 72 is rotatable in the circumferential direction C, vertical direction and horizontal direction. It is possible for an operator easily to manipulate the endoscope, because effects similar to the self-propelled type of guide assembly 145 of Fig. 9 are obtained, and movement of the elongated tube 13 in the vertical and horizontal directions can be considered.

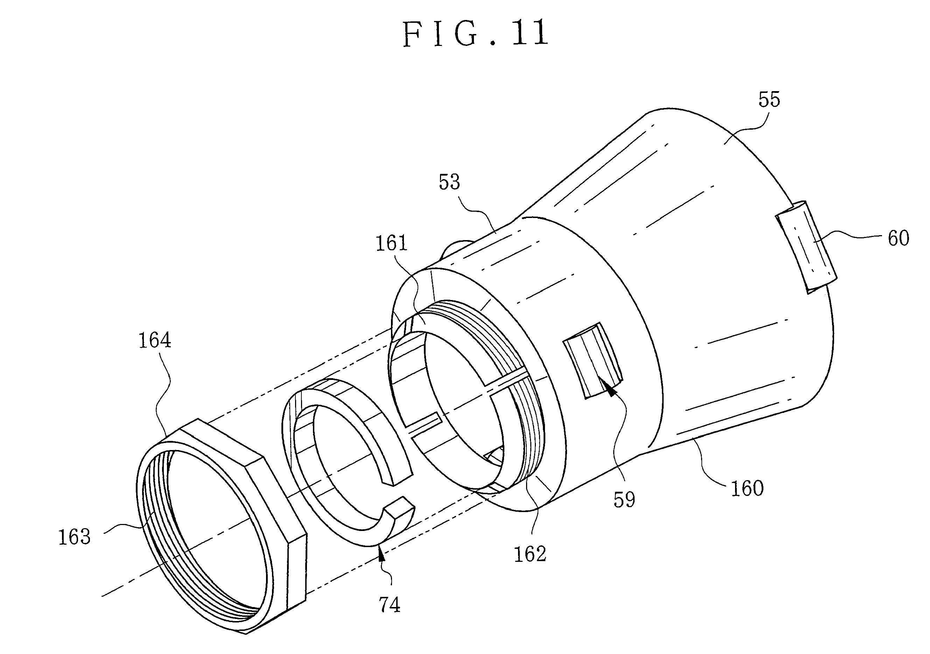

A retaining element other than the rubber ring 77 can be also used in the invention. In Fig. 11, one preferred second support barrel 160 is illustrated. A collet portion 161 or collet chuck device with plural collet segments protrudes from the second support barrel 160. Male threads 162 are formed about the collet portion 161. A nut 164 is used, and includes female threads 163 helically engaged with the male threads 162 for retention to the collet portion 161. This is effective in firmly retaining the elongated tube 13 to the second support barrel 160. The collet segments in the collet portion 161 are a collet chuck device, which has an inclined surface with an inclination in an inward direction toward the distal side. Also, an inner surface of the nut 164 is inclined to decrease its diameter in the distal direction according to the collet portion 161. An interval between the collet segments of the collet portion 161 decreases according to degree of engagement of the nut 164 with the collet portion 161, to clamp the elongated tube 13 in the spacer 74 tightly. Remaining portions of the second support barrel 160 are the same as those of the second support barrel 51. Elements similar to those of the above embodiments are designated with identical reference numerals.

Also, various elements other than the rubber ring and nut can be used for the retention. Among those, a hose band clip or hose band can be used for adjusting a size of the ring with a screw. Also, a screw hole and a screw can be used. The screw hole is formed in a retaining portion in a direction vertical to the axial direction A. The screw is helically engaged with and retained to the screw hole. In the use of the screw, it is unnecessary to use a collet chuck device for the retaining portion.

A spacer according to the invention may have a form other than the C ring described above, for example, a combination of plural ring segments defined by splitting a ring. Also, a spacer for use may have a form similar to the retaining device 72, namely, may be a recessed ring having a ring portion and plural recesses formed in the ring portion to open in the axial direction.

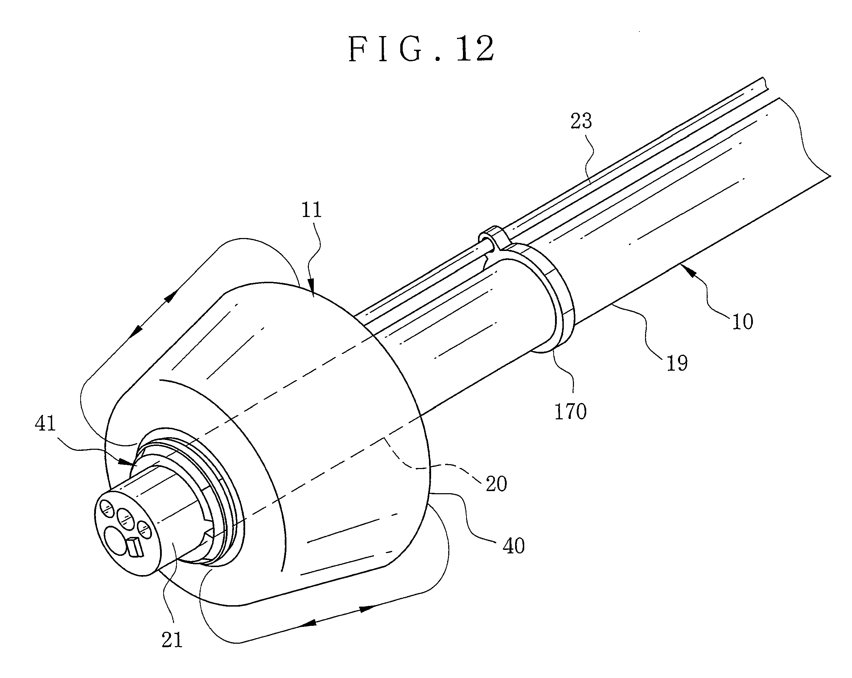

In the above embodiments, the overtube 24 is used to cover the insertion tube, and the protection sheath 23 is inserted in the overtube 24. However, a guide ring 170 of Fig. 12 can be used in place of the overtube 24. The elongated tube 13 and the protection sheath 23 are retained by use of the guide ring 170. This is effective in preventing enlargement of a diameter of the elongated tube 13 because of absence of the overtube.

The end cup, although shaped conically in the above embodiment, may be shaped semi-spherically or in other forms.

In the above embodiment, the end cup has the gradually increasing diameter. However, an end cup of the invention may be a simple portion having a larger diameter than the barrel sleeve, for example, can include a first cup portion of a cylindrical shape and a second cup portion formed between the first cup portion and the barrel sleeve with an inclination of an increasing diameter according to a difference in the diameters of the first cup portion and the barrel sleeve. Furthermore, the end cup may have an intermittent shape without a continuous surface, for example, may be a combination of plural rods or strips extending in an arrangement of a cup or frustum of a cone, except for continuous areas located for the drive roller and the idler roller.

Also, the end cup or skirt can be formed from flexible material. An actuator, such as a piezoelectric device or shape memory alloy, may be combined and driven to shift the end cup for enlargement and reduction. The end cup may be set in a closed position for entry of the insertion tube into a body cavity, and set in an open position for steering of the steering portion. Effects similar to the above embodiments can be obtained similarly.

In the above embodiments, the self-propelled type of guide assembly is used with the endoscope for a medical use. Also, the guide assembly of the invention can be used with an endoscope for industrial use, an ultrasonic probe, or other instruments for imaging in a cavity. Although the movable endless track device or crawler device or toroidal device is turned around in the guide assembly, a guide assembly of the invention can be any mechanical type for entry in a body cavity as a component for an instrument for imaging.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A guide assembly for an endoscope (10) having an elongated tube (13) for entry in a body cavity, comprising:
a barrel (50, 51, 91, 92, 111, 112, 126, 127), having a lumen (71) for receiving said elongated tube in an axial direction (A) thereof; and
a retaining device (72, 77, 104) for retaining said barrel to said elongated tube to provide a clearance space (45, 46) defined between said barrel and said elongated tube.

2. A guide assembly as defined in claim 1, wherein said elongated tube includes a head assembly, a flexible device, and a steering device disposed between said head assembly and said flexible device;
said retaining device is secured to one of said head assembly, said steering device, and a connection point between said steering device and said flexible device.

3. A guide assembly as defined in claim 2, wherein said barrel includes:
a barrel sleeve, having a first inner diameter according to an outer diameter of said elongated tube; and
at least one large diameter portion, formed to extend from an end of said barrel sleeve, having a second inner diameter larger than said first inner diameter, for allowing said steering device in said elongated tube to steer.

4. A guide assembly as defined in claim 3, wherein said large diameter portion is formed to extend from at least one of distal and proximal ends of said barrel sleeve in said axial direction.

5. A guide assembly as defined in claim 4, wherein said second inner diameter in said large diameter portion increases in a direction away from said barrel sleeve.

6. A guide assembly as defined in claim 4, wherein said at least one large diameter portion includes:
a first skirt formed to extend from said distal end of said barrel sleeve; and
a second skirt formed to extend from said proximal end of said barrel sleeve.

7. A guide assembly as defined in claim 3, further comprising a bearing structure, disposed between said barrel and said retaining device, for connection thereof in a relatively rotatable manner.

8. A guide assembly as defined in claim 7, wherein said bearing structure includes a bearing ball.

9. A guide assembly as defined in claim 7, wherein said bearing structure includes:
a bearing support ring disposed between said barrel and said retaining device;
a first bearing ball disposed between said barrel and said bearing support ring; and
a second bearing ball disposed between said retaining device and said bearing support ring.

10. A guide assembly as defined in claim 3, further comprising a spacer, contained between said retaining device and said elongated tube, for maintaining said clearance space between said barrel and said elongated tube.

11. A guide assembly as defined in claim 10, wherein said spacer includes a spacer ring, and at least one gap formed in said spacer ring.

12. A guide assembly as defined in claim 11, wherein said spacer is shaped in a C form.

13. A guide assembly as defined in claim 3, further comprising a collet chuck device formed with a distal end of said barrel;
wherein said retaining device is fitted around said collet chuck device for retention.

14. A guide assembly as defined in claim 13, wherein said retaining device is an elastic retaining ring.

15. A guide assembly as defined in claim 13, wherein said retaining device includes:
a male thread formed around said collet chuck device; and
a nut having a female thread engaged helically with said male thread and secured to said collet chuck device.

16. A guide assembly as defined in claim 3, further comprising:
a movable endless track device, having an annular surface, disposed to extend outside said barrel, movable on an endless track, for contacting a wall of a body cavity for propulsion in said axial direction;
a driving device for moving said endless track device on said endless track relative to said barrel.

17. A guide assembly as defined in claim 16, wherein said driving device includes:
a first drive roller, having engagement teeth, supported on said barrel, rotatable about an axis extending transversely to said axial direction, for moving said endless track device by rotation of said engagement teeth; and
a rotating mechanism for rotating said first drive roller.

18. A guide assembly as defined in claim 17, wherein said retaining device is disposed close to said first drive roller.

19. A guide assembly as defined in claim 17, wherein said rotating mechanism includes a first worm gear, disposed concentrically with said barrel in a rotatable manner, for rotating said first drive roller.

20. A guide assembly as defined in claim 19, wherein said rotating mechanism further includes a control wire, disposed to extend from said barrel in a proximal direction along said elongated tube, for transmitting driving force in a distal direction upon being rotated on a proximal side, to rotate said first worm gear.

21. A guide assembly as defined in claim 20, further comprising a guide portion, secured to said elongated tube, having a hole for receiving entry of said control wire, for guiding said control wire along said elongated tube.

22. A guide assembly as defined in claim 19, wherein said barrel includes:
a first support barrel, having said endless track, for supporting said endless track device movably;
a second support barrel, mounted in said first support barrel, and secured to said elongated tube with said retaining device.

23. A guide assembly as defined in claim 19, further comprising:
a second drive roller, having engagement teeth, disposed on a proximal side in said axial direction from said first drive roller, supported on said barrel, rotatable about an axis extending transversely to said axial direction, for moving said endless track device by rotation of said engagement teeth;
a second worm gear, formed with said first worm gear on said proximal side in said axial direction, for rotating said second drive roller.

24. A guide assembly as defined in claim 20, wherein said rotating mechanism further includes:
a pinion, disposed on a proximal side from said barrel, and rotated by a distal end of said control wire; and
a spur gear, formed at a proximal end of said first worm gear concentrically, and caused to rotate by said pinion upon rotation of said control wire.
